# EUROPEAN PATENT APPLICATION

(11) **EP 1 965 211 A1**
(43) Date of publication of application: **03.09.2008**
(21) Application number: 06834673.3
(22) Date of filing: 14.12.2006
(51) Int. Cl.: G01N 33/53, C08B 37/00

(54) **METHOD OF DETECTING SUGAR CHAIN HAVING GlcNAc TRANSFERRED BY GnT-V**

(30) Priority: 22.12.2005 JP 2005369426
(71) Applicant: J-Oil Mills, Inc., Tokyo 104-0044 (JP); National Institute of Advanced Industrial Science and Technology, Tokyo 100-8921 (JP)
(72) Inventor: HIRABAYASHI, Jun, Ibaraki 305-8568 (JP); NAKAMURA, Sachiko, Ibaraki 305-8568 (JP); KAMEI, Masugu, Tokyo 104-0044 (JP); KOMINAMI, Junko, Tokyo 104-0044 (JP)
(74) Representative: Hellmich, Wolfgang
(86) International application number: PCT/JP2006/324919
(87) International publication number: WO 2007/072724

(57) **Abstract**

It is intended to provide a method by which sugar chains having GlcNAc transferred by GnT-V can be accurately detected, screened and purified. In this detection method, two kinds of lectins differing in detailed GlcNAc-specificity are used together. As shown in Fig. 1(I), for example, a sample containing sugar chains having GlcNAc transferred by GnT-V is screened by using a lectin GSL-II, which recognizes sugar chains having GlcNAc transferred by GnT-V, and then the screened sample is screened again by using another lectin BLL, which does not recognizes sugar chains having GlcNAc transferred by GnT-V, to thereby detect, screen and purify the sugar chains having GlcNAc transferred by GnT-V alone.

## Description

### Technical Field

The present invention relates to a method of detecting sugar chains synthesized by N-acetylglucosamine transferase-V (hereinafter referred to as GnT-V), and more particularly to the method useful for accurately detecting sugar chains characteristic of metastatic cancer.

### Background Art

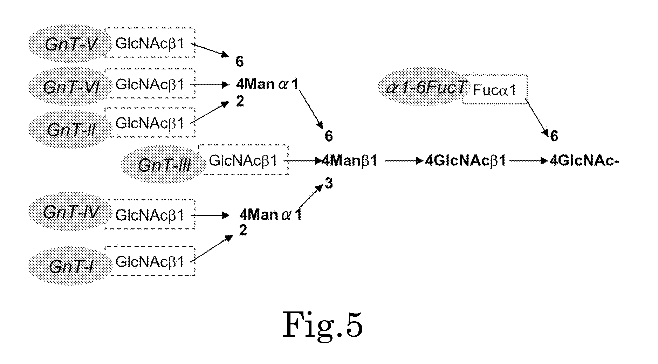

Examples of glycosyltransferases for determining branched-chain structure of an N-linked sugar chain of protein include N-acetylglucosamine transferase-III (GnT-III), -IV (GnT-IV), -V (GnT-V), -VI (GnT-V) and the like. As shown in Fig. 5, such N-acetylglucosamine transferases play a role of transferring and adding nonreducing terminal N-acetylglucosamine (GlcNAc) to each of specific branched chains. The sugar chains that have been added are involved in various vital functions.

For example, GnT-III syntheses a bisecting GlcNAc residue on N-linked sugar chain of complex or hybrid types. Dwek Ph. D. et al. in United Kingdom found that a BSE prion protein has less sugar chains to be added by GnT-III than a normal prion protein. Decreased expression of GnT-III gene can increase disease-related prion proteins. It is also suggested that GnT-III has the effect of suppressing cancer metastasis such as lung metastasis of melanoma, the effect of suppressing proliferation of hepatitis B viruses (HBV), the effect of suppressing secretion of HBV-related proteins and the effect of increasing the IgG activity.

GnT-V synthesizes a branch of GlcNAcß1-6Manα1-6 Manß1-4 on an N-linked sugar chain. Although the number of branched side chains in N-linked sugar chain is normally 3 to 4, the number will increase as an organ becomes cancerous or as T-lymphocytes become activated. An increase in the number of side chains leads to an increase in the amount of poly-N-acetyllactosamine known to be caused to increase by a cancer. It is known that metastasis of cancer is suppressed in a mouse in which a gene of N-acetylglucosamine transferase-V has been destroyed and that the GnT-V activity is enhanced in a highly metastatic cancer. In addition, it is pointed out that GnT-V is involved not only in cancer metastasis but also in carcinogenesis, since the expression of GnT-V increases in the early stage of canceration in the process of liver carcinogenesis.

### Disclosure of Invention

A sugar chain synthesized by GnT-V among N-acetylglucosamine transferases is particularly useful for life mechanism and treatment of disease as it plays a role of life information substance related to cancer metastasis. Accordingly, there is a demand for accurately identifying sugar chain having GlcNAc transferred by GnT-V

Lectin is a protein which specifically binds with sugar chain contained in glycoprotein or glycolipids on cell membrane surface. The lectin has an effect as follows: when a lectin recognizes sugar chain in a glycoprotein and binds with the sugar chain it will cause hemagglutination in the case where the cell is a blood cell and will cause cell agglutination in the case where the cell is a bacterium. Clinical diagnostic agents, reagents, therapeutic agents and the like have been developed, utilizing the specific recognition effect and the agglutination effect of lectin.

Wheat germ agglutinin, phaseolus vulgaris agglutinin and the like are known as lectins that specifically recognize sugar chains to which N-acetylglucosamine (GlcNAc) is added. Lectins are generally said to have specific binding properties to certain sugar chains. However, currently-known GlcNAc -specific lectins do not satisfy the above demand to accurately identify sugar chains synthesized by GnT-V since currently-known GlcNAc -specific lectins recognize not only sugar chains transferred by GnT-V but also sugar chains synthesized by GnT-III and GnT-VI. Therefore, it is difficult to accurately identify sugar chains having GlcNAc transferred by GnT-V with use of single lectin under the current circumstance.

The present invention provides methods for reliably and highly accurately detecting, purifying and screening sugar chain having GlcNAc transferred by GnT-V

The present inventors devoted themselves to study the problem described above. As a result of the study, they found that there are two types in GlcNAc -specific lectins: a type of lectin that recognizes sugar chain having GlcNAc transferred by GnT-V and a type of lectin that does not recognize such sugar chain and that combined use of these two types of lectins can solve the above problem. That is, the method of detecting sugar chain having GlcNAc transferred by GnT-V according to the invention is characterized in that at least a kind of GlcNAc -specific lectins having affinity for sugar chain having GlcNAc transferred by GnT-V and at least a kind of GlcNAc -specific lectins having no affinity for sugar chain having GlcNAc transferred by GnT-V are used in combination. In the detection method of the invention, it is important to use two types of different GlcNAc -specific lectins. These lectins may be used simultaneously or they may be used in time series, in other words, in a two-stage operation. In the case of two-stage operation, any one of lectins may precede.

In the present specification, the term "sugar chain" is used so as to include all of free oligosaccharides, sugar chains fluorescent-labeled with Cy3, aminopyridine or the like, glycoamino acids, glycopeptides, glycoproteins, proteoglycans, cells and the like, as long as the sugar chains are N-linked sugar chains. Glycoproteins include those of high-mannose type, hybrid type, complex type and the like. In addition, a substance obtained partially degrading the above sugar chain with a single use, a combined use or a use in a sequential manner of sialidase, galactosidase, N-acetylhexosaminidase and fucosidase may be used.

Said GlcNAc -specific lectin having no affinity for sugar chains having GlcNAc transferred by GnT-V preferably have affinity for sugar chains having GlcNAc transferred by GnT-I and/or GnT-II. Said GlcNAc -specific lectin having affinity for sugar chains having GlcNAc transferred by GnT-V is at least one type of lectins selected from a group consisting of, for example, GSL-II, DSA, EVA, ECA, LEA, PWM, STA, WGA, PVL, PHA and CEL-I, and said GlcNAc -specific lectin having no affinity for sugar chains having GlcNAc transferred by GnT-V is at least one lectin selected from BLL and ABA.

Said sugar chain having GlcNAc transferred by GnT-V is, for example, sugar chain which is characteristic of a metastatic cancer.

The invention further provides a method of purifying sugar chains having GlcNAc transferred by GnT-V, wherein at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V are used in combination. The invention further provides sugar chains having GlcNAc transferred by GnT-V purified by the above purification method.

The invention further provides a method of screening sugar chains having GlcNAc transferred by GnT-V, wherein at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V are used in combination.

The invention further provides a detection reagent kit for detecting sugar chains having GlcNAc transferred by GnT-V, wherein the detection reagent kit contains at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V

The invention also provides a kit for diagnosing a disease derived from sugar chains having GlcNAc transferred by GnT-V or a disease for which the sugar chain plays a role of marker, wherein the diagnostic agent kit contains the detection reagent kit containing at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V.

The invention further provides a method of determining affinity for sugar chains having GlcNAc transferred by GnT-V of a GlcNAc -specific lectin with use of sugar chains having GlcNAc transferred by GnT-V detected by the above method. The method is useful for selecting lectins having no affinity for sugar chains having GlcNAc transferred by GnT-V from those called as GlcNAc -specific lectins.

The invention enables accurate detection, screening and purification of sugar chains having GlcNAc transferred by GnT-V through combined use of two types of lectins differing in detailed GlcNAc -specificity.

Said sugar chains having GlcNAc transferred by GnT-V is also characteristic of a metastatic cancer. Accordingly, the invention enables easy and accurate diagnosis of cancer metastatic property and improves the diagnostic yield. The invention can also be used for determining the effect and the prognosis of treatment of a cancer.

### Brief Description of the Drawings

[Fig. 1] Fig. 1 is a flow sheet of detection processes (I) and (II) according to the preset invention.
[Fig. 2A] Fig. 2A is a structural visualization of PA-oligosaccharide used in the detection method of the invention. The reducing terminal has been pyridylaminated.
[Fig. 2B] Fig. 2B is a structural visualization of PA-oligosaccharide (which is continued from Fig. 2A) used in the detection method of the invention. The reducing terminal has been pyridylaminated. Symbols used for indicating a monosaccharide pyranose ring and symbols for indicating whether the type of the linkage is α or ß are shown in the frame below the chart.
[Fig. 3] Fig. 3 is a bar chart showing binding constants (Kₐ) of (A) pNP-sugar and (B) PA-sugar for GSL-II (on the left side) and BLL (on the right side).
[Fig. 4] Fig. 4 is a bar chart showing binding constants (Kₐ) of (A) GSL-II and (B) BLL with partial or complete agalacto N-linked sugar chains. In the chart, sugar chains are aligned in the descending order of the binding constant.
[Fig. 5] Fig. 5 is a schematic diagram showing locations at which GlcNAc is transferred by various N-acetylglucosamine transferases.

### Best Mode for Carrying Out the Invention

An embodiment of the invention will now be described with reference to the attachment drawings. A sugar chain having GlcNAc transferred by GnT-V to be detected by the detection method of the invention is expressed, for example, in the following chemical formula: 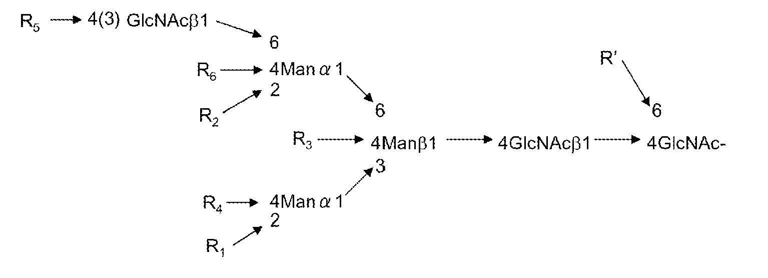 wherein R' is OH or α-L-fucose; each R₁, R₂, R₃, R₄ and R₆ is independently OH or a sugar residue, for example, GlcNAcß1, Galß1-3GlcNAcß1, Galß1-4GlcNAcß1, Siaα2-3 Galß1-3GlcNAcß1, Siaα2-3Galß1-4GlcNAcß1, Siaα2-6Galß1-3GlcNAcß1, Siaα2-6Galß1-4GlcNAcß1, or partial repetition thereof, sugar chains attached with fucose, such as Siaα2-6Galß1-4GlcNAc ß1-6Galß1, Fucα1-2Galß1 or the like; and R₅ is OH or a sugar residue, such as Galß1, Siaα2-3Galß1, Siaα2-6Galß1 in general, sugar chains with a repetitive lactosamine structure beyond Gal, or sugar chains partially attached with α-L-fucose, such as Siaα2-6Galß1-4GlcNAcß1-3Galß1. In addition, the sugar chains having GlcNAc transferred by GnT-V also include sugar chain in which α-L-fucose is attached to either one of 2,3,4,6-position hydroxyl groups of GlcNAc transferred by GnT-V

The detection method of the invention requires a combined use of at least one type of GlcNAc -specific lectins having affinity for sugar chains having GlcNAc transferred by GnT-V, and at least one type of GlcNAc -specific lectins having no affinity for sugar chains having GlcNAc transferred by GnT-V. The materials will now be described.

Firstly, GlcNAc -specific lectins having affinity for sugar chains having GlcNAc transferred by GnT-V may be of any origin such as of animals, plants, fungi, bacteria and virus, as long as the GlcNAc -specific lectins have specific affinity. Furthermore, GlcNAc -specific lectins may be a variant of polypeptide substantially homologous to natural lectin having a different amino-acid sequence from a natural lectin caused by deletion, insertion or substitution, or may be an artificial polypeptide as long as GlcNAc -specific lectins retain the above described property.

Specific examples of lectins of natural ingredients include Griffonia simplicifolia lectin II (GSL-II), Datura stramonium agglutinin (DSA), Erythrina variegate agglutinin (EVA) and Erythrina cristagalli agglutinin (ECA), Lycopersicon esculentum agglutinin (LEA), Pokeweed mitogen lectin (PWM), Solanum tuberosum agglutinin (STA), wheat germ agglutinin (WGA), Psathyrella velutina lectin (PVL), Phaseolus vulgaris agglutinin (PHA) and Cucumaria echinata lectin (CEL-I).

With regard to affinity for sugar chains having GlcNAc transferred by GnT-V, the binding constant (Kₐ) of lectins is normally 1.0 X 10³ M⁻¹ or more, preferably 1.0 X 10⁴ M⁻¹ or more, and particularly preferably 1.0 X 10⁵ M⁻¹ or more.

Secondly, GlcNAc -specific lectins having no affinity for sugar chains having GlcNAc transferred by GnT-V may be of any origin such as animals, plants, fungi, bacteria and virus. Furthermore, GlcNAc -specific lectins may be a variant of polypeptide substantially homologous to natural lectin having a different amino-acid sequence from a natural lectin caused by deletion, insertion or substitution, or may be an artificial polypeptide as long as GlcNAc -specific lectins retain the above described property.

The lectin is obtained by, for example, searching a lectin having affinity for sugar chains having GlcNAc transferred by GnT-I and/or GnT-II from GlcNAc -specific lectins. Examples of lectins of natural orign include Boletopsis leucomelas lectin (BLL) and Agaricus bisporus agglutinin (ABA).

With regard to no affinity for sugar chains having GlcNAc transferred by GnT-V, the binding constant (Kₐ) of lectins is normally less than 1.0 X 10³ M⁻¹, preferably less than 1.0 X 10² M⁻¹, and particularly preferably less than 1.0 X 10¹ M⁻¹.

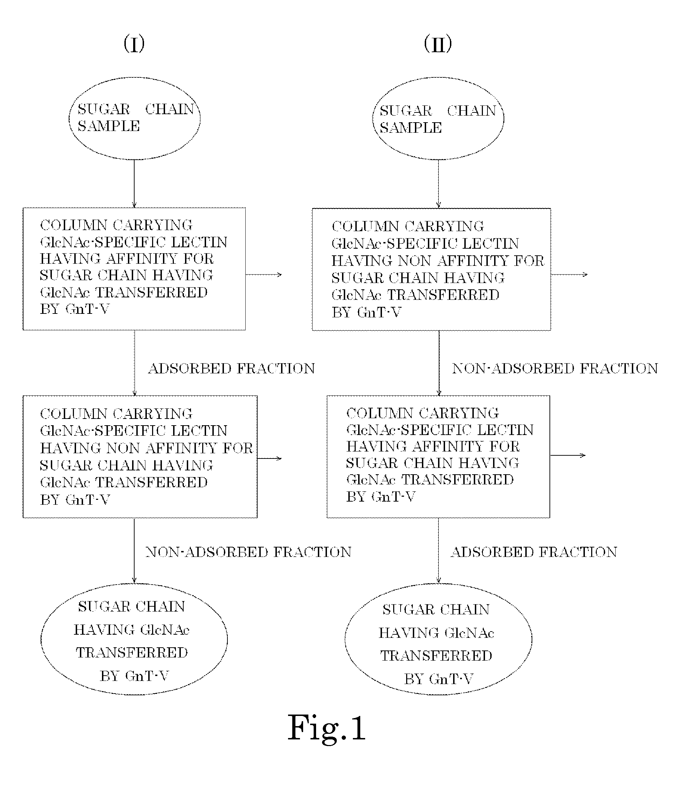

The detection method of the invention will now be described with reference to Fig. 1(I). First, a sugar chain sample is made to be adsorbed with use of a lectin that recognizes sugar chains having GlcNAc transferred by GnT-V, and subsequently the adsorbed sugar chains are rescreened with use of a lectin that does not recognize sugar chain having GlcNAc transferred by GnT-V. Sugar chain having GlcNAc transferred by GnT-V is accurately detected from the second non-adsorbed fraction.

In the above detection method, as shown in Fig. 1(II), sugar chain sample may be made to pass through a column onto which at least one type of GlcNAc -specific lectins having no affinity for sugar chain having GlcNAc transferred by GnT-V is immobilized, and the non-adsorbed fraction thereof may be made to pass through a column onto which at least one type of GlcNAc -specific lectins having affinity is immobilized. In the latter case, the non-adsorbed fraction in the first column is made to pass through the second column, and sugar chain having GlcNAc transferred by GnT-V is accurately detected from the adsorbed fraction thereof.

Lectin chromatography may be used for the detecting operation in the above various steps. The lectin chromatography is an affinity chromatography that uses the property of a lectin such that it specifically binds with sugar chains. When the lectin chromatography is combined with High-performance liquid chromatography (HPLC) (high-performance liquid affinity chromatography: HPLAC), a high throughput is expectable. Common carriers are gel materials such as agarose, dextran, cellulose, starch, and polyacrylamide. For carriers, commercially available products can be used without particular limitation, and examples thereof include Sepharose 4B and Sepharose 6B (manufactured by GE Healthcare Bioscience). In addition, columns used for the lectin chromatography include a column onto which a lectin is immobilized onto a microplate and nano well.

The concentration of a lectin to be immobilized is normally 0.001 to 20 mg/ml, preferably 0.01 to 10 mg/ml. In the case where an agarose gel is used as a carrier, the agarose gel is coupled with a lectin after it is activated by CNBr. A lectin may be immobilized to a gel into which an activated spacer has been introduced. Furthermore, a lectin may be reduced with NaCNBH₃ after the lectin is immobilized onto a gel into which a formyl group has been introduced. In addition, a commercially available activated gel such as NHS-Sepharose (manufactured by GE Healthcare Bioscience) may be used.

After the sugar chain sample is made to pass through the column, a buffer solution is made to flow therein for the purpose of washing and equilibration. Examples of buffer solutions include buffer solutions with a molar concentration 5 to 500 mM, preferably 10 to 100 mM, with pH 4.0 to 10.0, preferably 6.0 to 9.0, with NaCl content 0 to 0.5 M, preferably 0.1 to 0.2 M, with CaCl₂, MgCl₂ or MnCl₂ content 0 to 10 mM, preferably 1.0 to 5 mM.

After the affinity column is washed, sugar chains is eluted with use of a desorbent in a neutral nondenaturing buffer solution that enables effective elution of sugar chains. The buffer solution may be the same solution as described above. The desorbent may be N-acetylglucosamine (GlcNAc) or (GlcNAc)ₙ. The desorbent is GlcNAc with a concentration preferably 1 to 500 mM, particularly preferably 10 to 200 mM.

In the case where two types of lectins are used simultaneously as an alternative detection method of the invention, one of the lectins is labeled with Cy3 (green), and the other lectin is labeled with Cy5 (red). Examination on which color appears on the chip (microarray) or which color is deeper enables judging a degree of malignancy of a cancer.

It is preferable to use the frontal affinity chromatography (FAC) method for measuring the binding constant between lectin and sugar chain. The FAC method is based on the principle as follows: when a fluorescently-labeled sugar chains diluted solution at a certain concentration is made to flow through the column onto which a lectin is immobilized, if the lectin does not interact with the sugar chains, the sugar chains will flow out from the column within a short period of time, and thus an elution front end is immediately observed; and on the other hand, if the sugar chain has affinity for a lectin, elution of the sugar chain is delayed.

A lectin column to be used for the apparatus is prepared as follows:
1. Dissolve a purified lectin in 0.1 to 0.2 M NaHCO₃ buffer solution (pH 8.3 to 8.5);
2. Immobilize a lectin onto a carrier such as NHS-activated Sepharose via a primary amino group in a protein;
3. Suspend lectin-Sepharose in 10 mM Tris-HCl buffer solution (pH 7.4, TBS) containing 0.8% NaCl, and prepare a resin so that the lectin immobilizing concentration becomes 2 to 9 mg/ml;
4. Fill the lectin-immobilizing resin in a miniature column (φ 2 mm x 10 mm, 31.4 µl);
5. Sandwich a capsule with filters at the front and rear of the capsule; and
6. Protect the capsule filled with two types of immobilizing resins with a holder, and connect the lectin column to an FAC-1 system.

300µl each of pyridylaminated sugar chains (PA-sugar chains) is continuously poured into two buffered lectin columns at a flow rate of 0.125 ml/min. The PA-sugar chain has been diluted to the concentration (2.5 nM) which is sufficiently lower than the dissociation constant (K_{d}) of a lectin in a buffer solution for analysis (10 mM Tris-HCl buffer solution (pH 7.4) containing 0.8 % NaCl). Elution of the PA-sugar chain from the column is detected with use of a fluorescence detector (with exciting wavelength / fluorescence wavelength: 310 nm / 380 nm).

The delay (V-V₀) of the elution front end (V) of each sugar chain interacting with the lectin is calculated from the detected data based on the elution front end (V₀) of a sugar chain(PA-rhamnose) that does not interact with the lectin as the control. Then, the binding constant (Kₐ) between the sugar chain and the lectin is determined from V-V₀ and Bt based on the FAC reference equation.

A chromatography other than the above, lectin chip, enzyme immunoassay method (ELISA), agglutination method, and the BiaCore (trademark) system may also be used for detecting sugar chain in a method known to those skilled in the art.

The invention further provides a method of purifying sugar chains having GlcNAc transferred by GnT-V, wherein at least a kind of GlcNAc -specific lectins having affinity for sugar chains having GlcNAc transferred by GnT-V, and at least a kind of GlcNAc -specific lectins having no affinity for sugar chains having GlcNAc transferred by GnT-V are used in combination.

In the case where a glycoprotein is purified with use of lectin column, an affinity column is prepared by covalently-bonding at least one type of GlcNAc -specific lectins having affinity for sugar chains having GlcNAc transferred by GnT-V with a column carrier made of agarose and cellulose via a functional group, and a sugar chain sample is made to pass through the column. If a substance interacting with a GlcNAc -specific lectin exists in the sugar chain sample, it is possible to carry out crude separation of the glycoprotein to be purified. Subsequently, an affinity column is prepared by covalently-bonding at least one type of GlcNAc -specific lectins having no affinity for sugar chains having GlcNAc transferred by GnT-V with a column carrier made of agarose and cellulose via a functional group, and the eluate is made to pass through the column, whereby sugar chains having GlcNAc transferred by GnT-V can be purified.

In the above purification method, the sugar chain sample may first be made to pass through a column onto which at least one type of GlcNAc -specific lectins having affinity for sugar chains having GlcNAc transferred by GnT-V is immobilized, and then the non-adsorbed fraction thereof may be made to pass through a column onto which at least one type of GlcNAc -specific lectins having affinity is immobilized.

The invention further provides sugar chains having GlcNAc transferred by GnT-V obtained by the above purification method. The purity of sugar chains (that is, the ratio of sugar chains having GlcNAc transferred by GnT-V with respect to sugar chains having GlcNAc synthesized by all GnTs) is normally 70 % or more, preferably 80 % or more, more preferably 90 % or more, and further preferably 95 % or more.

The invention further provides a method of screening sugar chains having GlcNAc transferred by GnT-V, wherein at least a kind of GlcNAc -specific lectins having affinity for sugar chains having GlcNAc transferred by GnT-V, and at least a kind of GlcNAc -specific lectins having no affinity for sugar chains having GlcNAc transferred by GnT-V are used in combination.

Said method includes that a specimen is made to pass through a column onto which at least one type of GlcNAc -specific lectins having affinity for sugar chains having GlcNAc transferred by GnT-V is immobilized, and the eluate thereof is made to pass through a column onto which at least one type of GlcNAc -specific lectins having no affinity is immobilized.

In the above method, a specimen may be first made to pass through a column onto which at least one type of GlcNAc -specific lectins having no affinity for sugar chains having GlcNAc transferred by GnT-V is immobilized, and the eluate thereof may be made to pass through a column onto which at least one type of GlcNAc -specific lectins having affinity is immobilized.

### Diagnostic agents

The invention further provides a diagnostic agent kit for diagnosing a disease derived from sugar chains by detecting sugar chains synthesized by a GnT-V transferase. Diseases derived from sugar chains synthesized by a GnT-V transferase include, for example, carcinogenesis, cancer metastasis, infiltration and malignant alteration. Specific examples of cancers include prostate cancer, breast cancer, stomach cancer, small intestinal cancer, colon cancer, colorectal cancer, renal cell cancer, pancreatic cancer, small cell lung cancer, non small cell cancer, uterus cancer, ovary cancer, thyroid cancer, soft-tissue sarcoma, bone sarcoma, melanoma, glioblastoma, astrocytoma, medulloblastoma, acute lymphoma, malignant lymphoma, Hodgkin's disease, non-Hodgkin's disease, acute myelocytic leukemia, chronic lymphatic leukemia and the like. The invention is particularly useful for cancer of poorly differentiated type.

The method of using a diagnostic agent kit shall be hereinafter described. First, a cancer tissue, or body fluid such as blood, blood serum, blood plasma, urine, saliva is collected from a patient. The collected cancer tissue is fractured according to the conventional method. An organic solvent is added to each sample to remove lipid. Subsequently, a glycoprotein is extracted by solubilization of membrane protein and glycoprotein with use of a surface acting agent. For example, chloroform-methanol, hexane-isopropane and the like are used as the above organic solvent. For example, octyl-ß-glycoside, octylthio-ß-glycoside, n-octyl-ß-D-glucopyranoside, n-heptyl-ß-D-thioglucopyranoside, 3-[(3-chloramid propyl)-dimethyl-ammonio]-1-propane sulfonate, 3-[(3-chloramid propyl)-dimethyl-ammonio]-2-hydroxy-1-propanesulfonate, dodecyl sodium sulfate, dodecyl lithium sulfate, sodium cholate and the like are used as the above surface acting agents.

Acetone or ammonium sulfate in an appropriate amount may be added to the above solubilized glycoprotein, so that the glycoprotein in the precipitate may be recovered after centrifugal separation. The extract containing the thus acquired glycoprotein is subject to an affinity column chromatography of a two-stage operation with use of the diagnostic agent kit of the invention. Detection of sugar chains having GlcNAc according to the present kit means existing of a sugar chain to which GlcNAc is transferred by GnT-V Therefore, it is highly likely that cancer tissue is metastatic.

The diagnostic agent kit also enables diagnosing prognosis of treatment of cancer qualitatively or quantitatively. In addition, the diagnostic agent kit is also applicable for searching a substance suppressing gene expression of GnT-V.

When a mammal or the like is immunized with a glycoprotein concentrated and purified by the above chromatography, an antibody that recognizes sugar chains having GlcNAc synthesized by GnT-V is obtained. A monoclonal antibody may be acquired as appropriately. Use of the antibody also enables detecting with a high sensitivity a metastatic cancer that exists in a blood serum or the like in the patient. As a result, the antibody is helpful for diagnosing a cancer metastasis or the like. The antibody can also be used as an antibody pharmaceutical for treating a cancer.

Determination method of GlcNAc -specific lectins The invention further provides a method for determining affinity of lectin for sugar chains having GlcNAc transferred by GnT-V with use of sugar chains having GlcNAc transferred by GnT-V The method is useful for selecting lectins having no affinity for sugar chains having GlcNAc transferred by GnT-V from those called GlcNAc -specific lectins.

Specifically, a test lectin that has been known to be specific to GlcNAc but the details thereof have not been known are prepared. An affinity column onto which a glycoprotein is immobilized to a carrier as ligand is prepared. The glycoprotein has been confirmed to have N-linked sugar chains (such as 107, 315, 411, 414, 108, 205, 318, 320, 316, 413) having a branch V that were used in an Example 1 to be described later and sugar chains synthesized by GnT-V Said test lectin is made to pass through the column, and an adsorbed component is eluted with use of a hapten sugar such as GlcNAc as desorbent.

A non-adsorbed fraction in the chromatography and the eluted fraction showing a weak interaction with the ligand are lectins having no affinity for sugar chains having GlcNAc transferred by GnT-V On the other hand, a fraction showing a strong interaction is evaluated as a lectin having affinity for sugar chains having GlcNAc transferred by GnT-V

The invention will be hereinafter described in details with reference to an Example. However, the invention is not limited to the following Example.

### [Example 1]

### [Preparation of oligosaccharides]

Galß- p-nitrophenyl (pNP), GalNAcß- pNP, Mana- pNP, GlcNAcα- pNP, Fucα- pNP and Galß1-4Glcß- pNP were purchased from Sigma Corporation (Saint Louis, Missouri, USA), and Gala- pNP, GalNAcα- pNP, Man6-pNP, Galß1-4 GlcNAcß-pNP, Galß1-3 GalNAcα-pNP (Core1), Galß1-3 (GlcNAc 61-6) GalNAcα-pNP- (Core2), GlcNAcß1-3GalNAcα-pNP (Core3) and GlcNAcß1-6GalNAcα-pNP (Core6) were purchased from Toronto Research Chemicals Inc. (North York, Canada), and Glc-α was purchased from Galbiochem (San Diego, California, USA). Other pNP glycosides (Glc6-pNP, GlcNAcß-pNP and (GlcNAcß1-4)₂₋₅α-pNP) were purchased from Seikagaku Corporation.

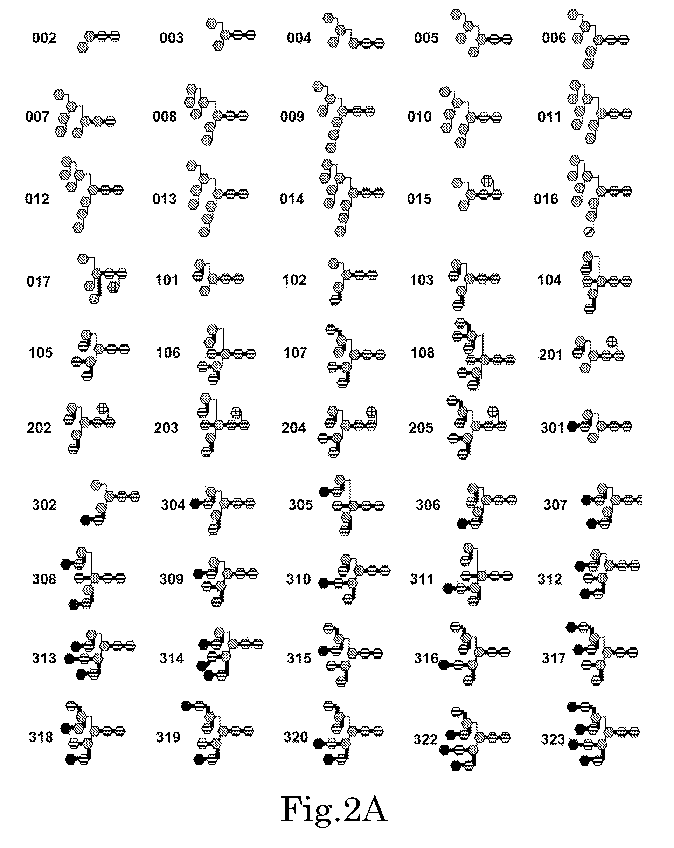

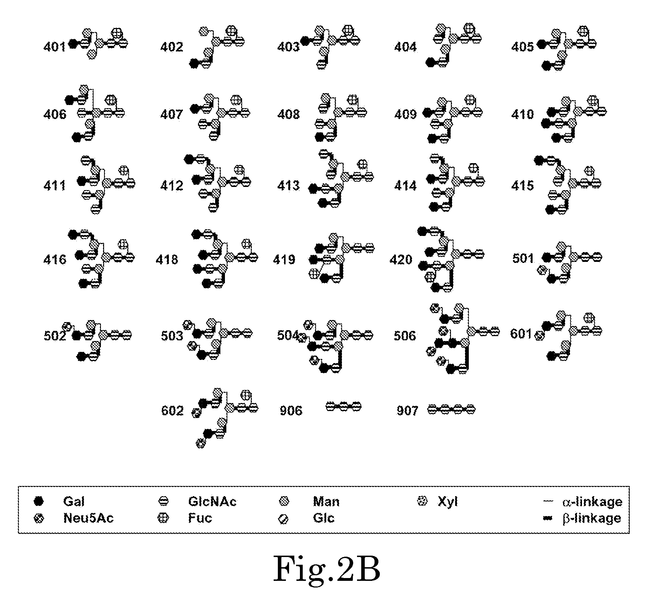

Pyridylaminated (PA-) oligosaccharides used in the Example are listed in Figs. 2A and 2B. N-linked sugar chains with numbers 001 to 014, 103, 105, 107, 108, 307, 313, 314, 323, 405, 410, 418 to 420, and 503 were purchased from Takara Bio Inc., and the other oligosaccharides were purchased from Seikagaku Corporation. Non-labeled oligosaccharides 906 and 907 were acquired from Seikagaku Corporation, and pyridylaminated with use of GlycoTAG (trademark, Takara Bio Inc.).

### [Preparation of lectins]

Griffonia simplicifolia lectins II (GSL-II)-agarose were acquired from Vector Laboratories (Burlingame, California, USA). Boletopsis leucomelas lectins (BLL) were acquired by purifying a fruit body of Boletopsis leucomelas, which is edible mushroom, by the method described in "Apoptosis induction by lectin isolated from the mushroom Boletopsis leucomelas in U937 cells," in Biosci. Biotechnol. Biochem. 66, 784-789.

### [Preparation of a lectin column]

BLL was dissolved in 0.2M NaHCO₃ buffer solution (pH 8.3) containing 0.5M NaCl, and bound with NHS-activated Sepharose (GE Healthcare Bioscience, Uppsala, Sweden) according to the operation manual of the manufacturer. The lectin-Sepharose was suspended in 10 mM Tris-HCl buffer solution (pH 7.4, TBS buffer solution) containing 0.8 % NaCl, and filled in a miniature column (φ 2 mm x 10 mm, 31.4 µl). A GSL-II lectin column was prepared in a similar manner.

### [Frontal affinity chromatography (FAC)]

A frontal affinity chromatography was carried out with use of an FAC automated analysis system (FAC-1, Shimadzu Corporation). More specifically, the lectin column prepared as described above was inserted in a stainless holder and connected to the FAC-1 system.

The flow rate and column temperature were kept at 0.125 ml/min and 25 °C, respectively. After the miniature column was equilibrated with the above TBS buffer solution, an excessive volume (0.5 to 0.8 ml) of a pNP-sugar chain (5µM) and a PA-sugar chain (2.5 or 5.0 nM) were continuously poured into the column with use of an automated sampling system.

Eluates of pNP-sugar chain and PA-sugar chain were monitored by measuring UV (280 nm) and fluorescence (with the exciting wavelength 310 nm and the fluorescence wave length 380 nm). A front end eluate (i.e., V-V₀) was measured with respect to a standard oligosaccharide (PA-lactose). In addition, the dissociation constant (K_{d}) was determined from V-V₀ and Bt based on the FAC reference equation. Note that the binding constant Kₐ is determined by the equation Kₐ = 1/K_{d}.

### [Analysis of the concentration dependency]

Analysis of the concentration dependency was carried out to measure the effective ligand concentration Bt. GlcNAcα-pNP at the initial concentration ([A]₀) was poured into the column for GSL-II, and Galß1-3GalNAcα-pNP at the initial concentration ([A]₀) was poured into the column for BLL. Next, V-V₀ (the delay of the eluted front end) was calculated in accordance with the method developed by Arata ("Application of reinforced frontal affinity chromatography and advanced processing procedure to the study of the binding property of a Caenorhabditis elegans galectin", J Chromatogr A. 905, 337-43). A Hofstee-type plot ((V-V₀) vs. (V-V₀) [A]₀) was created, and Bt and K_{d} were respectively determined from the intercept and the slope of the approximate curve.

### [Evalution of the lectin column with use of pNP-oligosaccharides]

BLL-agarose and GSL-II agarose were prepared at the concentrations 0.1 mg protein/ml gel and 3 mg protein/ml gel, respectively. Then, analysis of the concentration dependency was carried out to evaluate the column.

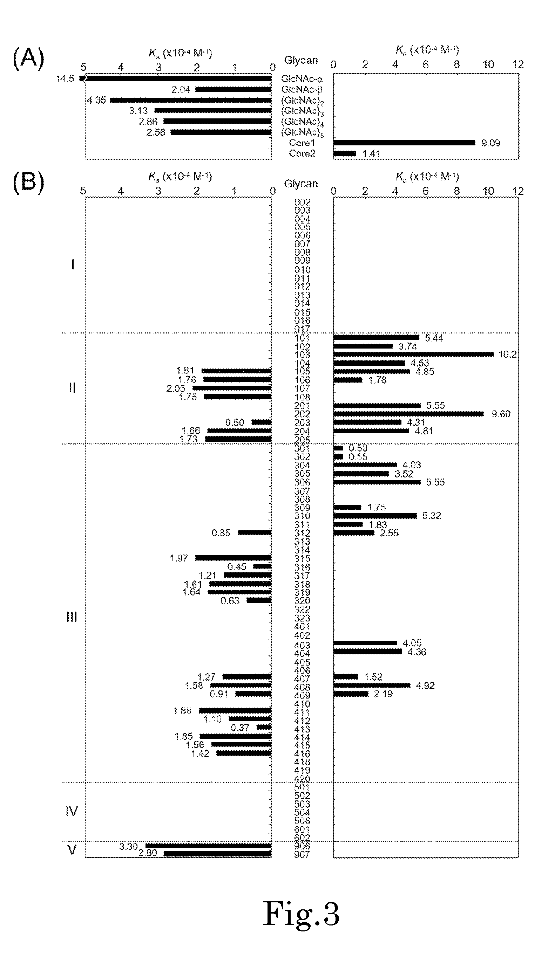

For GSL-II, GlcNAcα-pNP at various concentrations (2.5 to 70 µM) were prepared and poured into the column. As a result of the above FAC, Bt and Kₐ were respectively determined as 0.86 nmol and 1.4 x 10⁵ M⁻¹ (on the left side in Fig. 3(A)).

Although BLL was purified with GlcNAc -agarose, it did not show remarkable affinity for the tested GlcNAc -related pNP derivatives, that is, any one of GlcNAcα-pNP, GlcNAcß-pNP and (GlcNAc 61-4)₂₋₅α-pNP (on the right side in Fig. 3 (A)). BLL showed remarkable affinity for Galß1-3GalNAcα-pNP, and Bt and K_{d} were respectively 0.56 nmol and 9.09 x 10⁴ M⁻¹ (on the right side in Fig. 3(A)).

### [Evaluation of lectins with use of pNP-derivatives]

GSL-II showed the strongest affinity for GlcNAcα-pNP among the tested pNP glycosides, followed by chito-oligosaccharides, (GlcNAc ß1-4)₂α-pNP, (GlcNAcß1-4)₃α-pNP, (GlcNAcß1-4)₄α-pNP and (GlcNAcß1-4)₅α-pNP (on the left side in Fig. 3(A)). Although GSL-II also showed remarkable affinity for GlcNAcß-pNP, the binding was 1/7 of that of α-anomer.

BLL did not show detectable affinity for GlcNAcα/ß-pNP or chito-oligosaccharides (i.e., Kₐ < 3.4 x 10³ M⁻¹). BLL showed remarkable affinity for Galß1-3GalNAcα/ß-pNP (Core1) (Kₐ = 0.9 x 10⁵ M⁻¹).

### [Evaluation of lectins with use of PA-oligosaccharides]

Although GSL-II showed remarkable affinity for 105 to 108 and 203 to 205 among agalacto N-linked sugar chains 101 to 205 that contained no galactose, it did not show clear affinity for 101 to 104, 201 or 202 (on the left side in Fig. 3(B)). GSL-II showed strong affinity for 312, 315 to 320, 407 to 409, 411 to 416 among hybrid type N-linked sugar chains 301 to 420 to which galactose was completely or partially attached.

The Kₐ values measured by the FAC were processed into and shown in a bar chart to clarify the structural elements specifically recognized by GSL-II (on the left side in Fig. 3(B)). It is apparent from the chart that GSL-II binds strongly with tri- and tetra- antennary sugar chains, and it does not bind with monoantennary or biantennary sugar chains or slightly binds with them even if it does.

On the contrary, BLL recognized 101-106 and 201-204 with relatively high affinity but did not recognize 107, 108 or 205 at all among agalacto N-linked sugar chains 101-205 (on the right side in Fig. 3 (B)). Such selectivity was opposite to the case for GSL-II. Strong affinity was absorbed for 301 to 306, 309 to 312, 403, 404 and 407 to 409 among hybrid type N-linked sugar chains 301 to 420 to which galactose was completely or partially attached. This was also almost contrary to GSL-II.

As apparent from the right side in Fig. 3 (B), BLL shows the strongest affinity for agalacto biantennary N-linked sugar chains 103 and 202. While agalacto tri- antennary sugar chains 105 and 204 are also good ligand, the affinity therefor is substantially the same as the affinity for monoantennary sugar chains 101 and 201. No sugar chains having a branch V (GlcNAcB1-6 Manal-6ManB, on the right side in Fig. 3 (B)) has affinity for BLL. The invention is not limited by what is described below. However, since existence of a branch V generates a considerable change in a hybrid type N-linked sugar chain, recognition of BLL may require a steric configuration before the branch V of the agalacto biantennary N-linked sugar chain is attached.

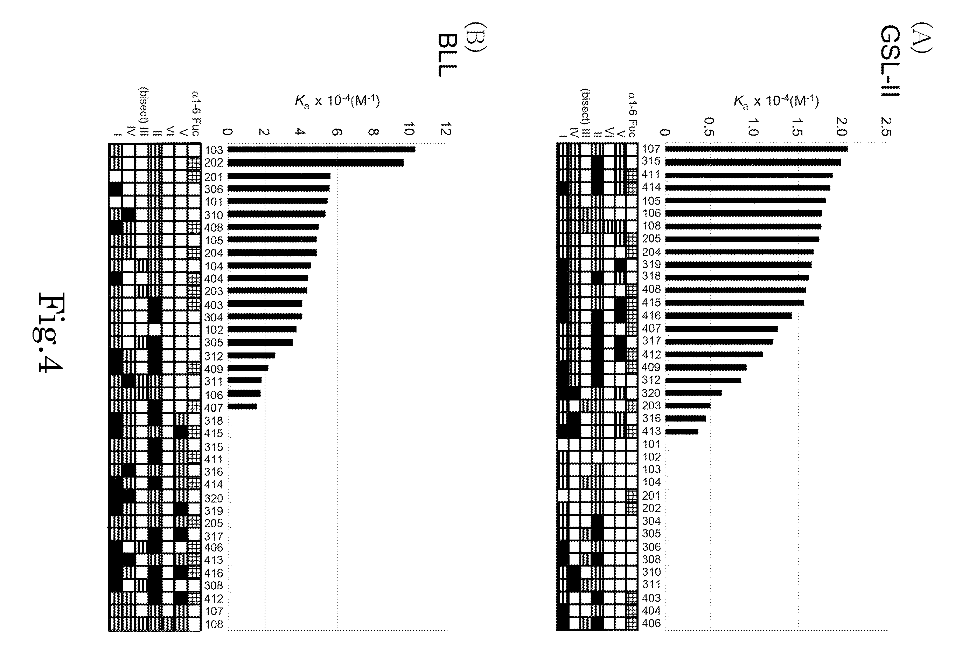

What has been described above will now be described referring to the branched chain map shown below the bar chart in Fig. 4. The map is created by categorization of GlcNAc branched chains the individual N-linked sugar chains have based on the nomenclature applied to human GlcNAc transferases. For example, if a section in V for a certain sugar chain has been filled, it means that the sugar chain has GlcNAc synthesized by a GnT-V enzyme.

When looking at the branched chain map in Fig. 4, GSL-II correctly recognizes N-linked sugar chains 107, 315, 411, 414, 108, 205 and the like having a branch V, they have GlcNAc transferred by GnT-V However, GSL-II also recognizes N-linked sugar chains 105, 106, 204, 408 and the like they do not have such a branch structure of the sugar chains. Therefore, single use of a lectin such as GSL-II which is said to recognize sugar chains having GlcNAc transferred by GnT-V cannot detect or purify only sugar chains having GlcNAc transferred by GnT-V

Although BLL does not recognize N-linked sugar chains 107, 315, 411 and the like containing sugar chains having GlcNAc transferred by GnT-V, it recognizes N-linked sugar chains 105, 106, 204, 408 and the like that do not have sugar chains having GlcNAc transferred by the GnT-V It is found from the both results that a combined use of GSL-II and BLL enables accurate detection of sugar chains having GlcNAc transferred by GnT-V What has been described above is summarized in Table 1.

**[Table 1]**

| | Sugar chains binding with BLL | Sugar chains not binding with BLL |
|---|---|---|
| Sugar chains binding with GSL-II | Group A 105 106 203 204 312 407 408 409 | Group B 107 108 205 315 316 317 318 319 320 411 412 413 414 415 416 |
| Sugar chains not binding with GSL-II | Group C 101 102 103 104 201 202 304 305 306 310 311 403 404 | Group D 308 406 |

The sugar chains with underlined sugar chain numbers in Table 1 are sugar chains having GlcNAc transferred by GnT-V Group B (group of sugar chains that bound with GSL-II but do not bind with BLL) contains all such sugar chains, and contains no sugar chains other than such sugar chains.

## Claims

1. A method of detecting sugar chains having GlcNAc transferred by GnT-V, wherein at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V are used in combination.

2. The method of detecting sugar chains having GlcNAc transferred by GnT-V according to claim 1, wherein the GlcNAc -specific lectin having no affinity for sugar chains having GlcNAc transferred by GnT-V has affinity for sugar chains having GlcNAc transferred by either or both of GnT-I and GnT-II.

3. The method of detecting sugar chains having GlcNAc transferred by GnT-V according to claim 1, wherein the GlcNAc -specific lectin having affinity for sugar chains having GlcNAc transferred by GnT-V is at least a kind of lectins selected from a group consisting of GSL-II, DSA, EVA, ECA, LEA, PWM, STA, WGA, PVL, PHA and CEL-I, and the GlcNAc -specific lectin having no affinity for sugar chains having GlcNAc transferred by GnT-V is at least a kind of lectins selected from BLL and ABA.

4. The method of detecting sugar chains having GlcNAc transferred by GnT-V according to claim 1, wherein the sugar chains having GlcNAc transferred by GnT-V are characteristics of metastatic cancers.

5. A method of purifying sugar chains having GlcNAc transferred by GnT-V, wherein at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V are used in combination.

6. The sugar chains having GlcNAc transferred by GnT-V purified by the method of purification according to claim 5.

7. A method of screening sugar chain having GlcNAc transferred by GnT-V, wherein at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V are used in combination.

8. A detection reagent kit for detecting sugar chains having GlcNAc transferred by GnT-V, wherein the detection reagent kit contains at least a kind of GlcNAc -specific lectins having affinity for sugar chains having GlcNAc transferred by GnT-V and at least a kind of GlcNAc -specific lectins having no affinity for sugar chains having GlcNAc transferred by GnT-V

9. A diagnostic agent kit for a disease derived from sugar chains having GlcNAc transferred by GnT-V or for a disease for which the sugar chains plays a role of a marker, wherein the diagnostic agent kit contains at least a kind of GlcNAc -specific lectins having affinity for sugar chain having GlcNAc transferred by GnT-V and at least a kind of GlcNAc -specific lectins having no affinity for sugar chains having GlcNAc transferred by GnT-V

10. A method of determining affinity for sugar chains having GlcNAc transferred by GnT-V of a GlcNAc -specific lectin with use of sugar chains having GlcNAc transferred by GnT-V detected by the method according to claim 1.
